(19) Europäisches Patentamt
European Patent Office
Office européen des brevets

(11) Veröffentlichungsnummer : **0 409 049 B1**

(12) # EUROPÄISCHE PATENTSCHRIFT

(45) Veröffentlichungstag der Patentschrift : **10.03.93 Patentblatt 93/10**

(21) Anmeldenummer : **90113144.1**

(22) Anmeldetag : **10.07.90**

(51) Int. Cl.[5] : **C07C 17/00,** C07C 25/24, C07C 41/18, C07C 43/225, C07C 43/29, C07D 405/06, C07D 303/08, C07D 521/00

(54) **Verfahren zur stereoselektiven Herstellung von Z-1,2-Diaryl-allyl-chloriden und deren Umsetzung zu Azolylmethyloxiranen sowie neue Zwischenprodukte.**

(30) Priorität : **18.07.89 DE 3923674**
**04.11.89 DE 3936823**

(43) Veröffentlichungstag der Anmeldung : **23.01.91 Patentblatt 91/04**

(45) Bekanntmachung des Hinweises auf die Patenterteilung : **10.03.93 Patentblatt 93/10**

(84) Benannte Vertragsstaaten : **AT BE CH DE DK ES FR GB GR IT LI NL SE**

(56) Entgegenhaltungen :
**EP-A- 0 196 038**
**DE-A- 2 652 313**
**GB-A- 2 146 987**
**US-A- 4 013 643**

(73) Patentinhaber : **BASF Aktiengesellschaft**
**Carl-Bosch-Strasse 38**
**W-6700 Ludwigshafen (DE)**

(72) Erfinder : **Kober, Reiner, Dr.**
**Im Schlittweg 20**
**W-6701 Fussgoenheim (DE)**
Erfinder : **Seele, Rainer, Dr.**
**Leiblstrasse 3**
**W-6701 Fussgoenheim (DE)**
Erfinder : **Isak, Heinz, Dr.**
**Schoenfelderstrasse 3**
**W-6704 Mutterstadt (DE)**
Erfinder : **Hickmann, Eckhard, Dr.**
**Kantstrasse 23**
**W-6701 Dannstadt-Schauernheim (DE)**
Erfinder : **Goetz, Norbert, Dr.**
**Schoefferstrasse 25**
**W-6520 Worms 1 (DE)**
Erfinder : **Zierke, Thomas, Dr.**
**Bahnhofstrasse 10**
**W-6737 Boehl-Iggelheim (DE)**

EP 0 409 049 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die stereoselektive Herstellung von Z-1,2-Diaryl-allylchloriden der allgemeinen Formel I

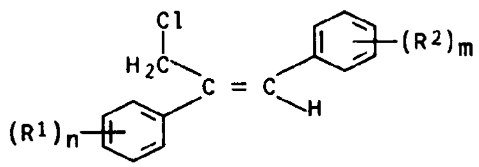

(I),

in der die Reste $R^1$ und $R^2$ unabhängig voneinander Wasserstoff, Halogen, Alkyl, Halogenalkyl, Alkoxi, Halogenalkoxi oder einen unsubstituierten oder substituierten aromatischen Rest bedeuten und n und m für 1, 2 oder 3 stehen.

Weiterhin betrifft die Erfindung die Umsetzung der Z-1,2-Diaryl-allylchloride zu Azolylmethyloxiranen der Formel IV

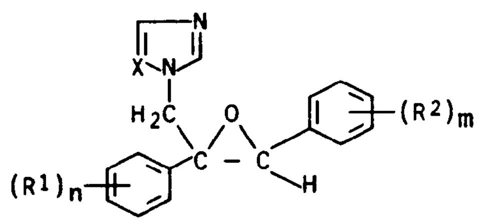

(IV),

in der die Reste $(R^1)_n$, $(R^2)_m$ die obengenannte Bedeutung haben und X für CH oder N steht.

Die neuen Zwischenprodukte I und die daraus hervorgehenden Epoxidationsprodukte V sind ebenfalls Gegenstand der Erfindung.

Verbindungen des Strukturtyps I sind gemäß den deutschen Offenlegungsschriften 32 18 129 und 32 18 130 sowie gemäß EP-A 196 038 und US-A-3 422 153 wertvolle Zwischenprodukte zur Herstellung von pharmakologischen, fungiziden und antimykotischen Wirkstoffen. Sie wurden bislang durch radikalische Halogenierung entsprechender Diarylpropenverbindungen (DE-A 32 18 129 oder EP-A 196 038) oder durch Oxidation und anschließende Substitution (DE-A 32 18 130) erhalten. Nachteilig an den Methoden des Standes der Technik sind die Verwendung teurer Reagenzien, z.B. teurer Halogenierungsreagenzien wie N-Bromsuccinimid für die radikalische Bromierung, die Anzahl der Synthesestufen und insbesondere die geringe Stereoselektivität.

Es ist allgemein bekannt, daß Moleküle, die in spezifischer Weise biologisch oder pharmakologisch wirken, in vielen Fällen definierte geometrische Anordnungen bestimmter funktioneller Gruppen besitzen müssen. Bei den fungiziden Wirkstoffen der allgemeinen Formel III bzw. IV (siehe DE-A 26 52 313) sind es vor allem die Z-konfigurierten Verbindungen (vgl. Sequenzregel nach Cahn, Ingold, Prelog), d.h. die Verbindungen, in denen die gegebenenfalls substituierten Phenylreste trans zueinander stehen, welche eine besonders hohe Wirksamkeit als Pflanzenschutzmittel besitzen.

Der Erfindung lag daher die Aufgabe zugrunde, ein Verfahren zu finden, nach welchem die Zwischenprodukte I in möglichst isomerenreiner Form, d.h. mit hoher Bevorzugung der E- bzw. trans-Konfiguration der Phenylreste an der Doppelbindung und in hoher Ausbeute hergestellt werden können. Weiterhin bestand die Aufgabe, unter Verwendung vorteilhafter Zwischenprodukte ein Herstellverfahren für die fungiziden Azolylmethyloxirane IV zu finden, das sich durch hohe Gesamtausbeuten und durch eine gegenüber den in DE-A 32 18 129 und 32 18 130 beschriebenen Verfahren verkürzte Anzahl von Reaktionsschritten auszeichnet.

Nach dem Stand der Technik lassen sich Aryl-substituierte Alkohole unter sauren Reaktionsbedingungen zum Beispiel unter Verwendung von Schwefelsäure in organischer Phase in die entsprechenden Aryl-substituierten Olefine bzw. Styrole überführen (s. z.B. Houben-Weyl, Methoden der organischen Chemie, 4. Auflage Band 5/1b -Alkene, Cycloalkene, Arylalkene, Georg Thieme Verlag Stuttgart, 1972, S. 62 ff, insbesondere S. 70 und 71; Tetrahedron, Band 26, S. 4277ff (1970).

Ebenso ist bekannt, daß derartige Reaktionen unter Zuhilfenahme von wasseraufnehmenden Reagenzien wie z.B. Acetanhydrid durchgeführt werden können. Allerdings sind für diese in der Literatur beschriebenen Eliminierungsreaktionen allgemein höhere Reaktionstemperaturen notwendig. Unter derartigen Reaktionsbedingungen erhält man nur unzureichende E-Z-Isomerenverhältnisse bezüglich der Aryl-Aryl-Anordnung.

Es wurde nun ein Verfahren zur stereoselektiven Herstellung von Z-1,2-Diaryl-allylchloriden der allgemei-

nen Formel I

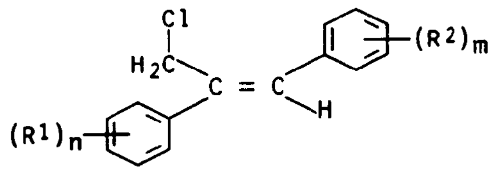

(I),

in der die Reste $R^1$ und $R^2$ unabhängig voneinander Wasserstoff, Halogen, Alkyl, Halogenalkyl Alkoxi, Halogenalkoxi oder einen substituierten aromatischen Rest bedeuten und n und m für 1, 2 oder 3 stehen, gefunden, das dadurch gekennzeichnet ist, daß man Chlorhydrine der Formel II

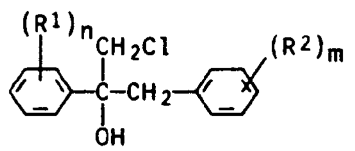

(II),

in der die Reste die o.g. Bedeutung haben, in einem inerten Ether oder Carbonsäureester als Lösungsmittel in Gegenwart eines Carbonsäureanhydrids und einer organischen oder anorganischen Säure bei Temperaturen bis 50°C dehydratisiert.

Nach dem erfindungsgemäßen Verfahren können Z-konfigurierte 1,2-Diarylallylchloride in hoher Stereoselektivität erhalten werden. Im allgemeinen und insbesondere nach den bevorzugten Ausführungsformen des Verfahrens liegen die Z:E-Verhältnisse bei 8:1 bis 15:1. Auch die hohe Regioselektivität mit der die Eliminierung von Wasser abläuft ist überraschend, denn man hätte in stärkerem Maße als Nebenreaktion auch eine Wasserabspaltung in Richtung der Chlormethyl-Seitenkette zu Chlorvinyldiarylverbindungen erwartet. Weiterhin können zu erwartende Konkurrenzreaktionen wie eine Substitution anstelle der Eliminierung erfolgreich unterdrückt werden. Ebenso findet eine erwartete Acylierung der Alkoholfunktion praktisch nicht statt.

Die Chlorhydrine der allgemeinen Formel II sind allgemein bekannt und können z.B. nach DE-A 28 51 086, EP-A 47 594 oder EP-A 15 757 in guten Ausbeuten durch Addition von Benzylgrignard-Verbindungen VI an ω-Chloracetophenone VII gemäß folgendem Reaktionsschema hergestellt werden:

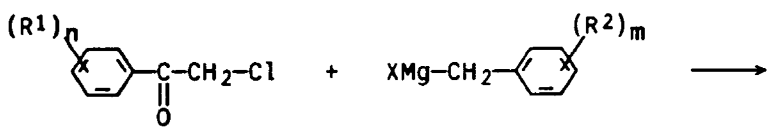

II

VII VI

$(X = Cl, Br)$

Bezüglich des Herstellungsverfahrens der Z-Allyl-chloride ist es ebenso vorteilhaft, zunächst die Chlorhydrine in Diethylether herzustellen und durch Zugabe von anorg. Säure, wie z.B. konz. Schwefelsäure, und Carbonsäureanhydrid zu der Diethyletherlösung bei ca. -10 bis 0°C die Dehydratisierung im Sinne eines Eintopfverfahrens durchzuführen.

Ebenso ist es möglich anstelle einer wäßrigen Aufarbeitung bei der Chlorhydrinsynthese, das Chlorhydrin durch Zugabe von äquimolaren Mengen an Säure, wie z.B. Schwefelsäure, aus der Magnesium-alkoxylat-Vorstufe freizusetzen und anschließend die Dehydratisierung durchzuführen.

Vorteilhaft und erfindungsgemäß ist dabei das allmähliche Zudosieren von Carbonsäureanhydrid, wobei die O-Acylierung von Chlorhydrin gegenüber der Dehydratisierung weitgehend unterdrückt werden kann.

Die erfindungsgemäße Dehydratisierung der Chlorhydrine II erfolgt in einem Ether oder Ester als Lösungsmittel. Im Fall offenkettiger Ether sind dabei solche mit zumindest 2 Sauerstoffatomen wie Ether von Glycolen und niedermolekularen aliphatischen Alkoholen, z.B. Ethylenglycoldimethyl- oder -diethylether bevorzugt. Besonders vorteilhaft sind cyclische Ether wie Tetrahydrofuran (THF) und insbesondere Dioxan. Geringe Zusätze aprotischer Lösungsmittel wie Essigester, halogenierte Kohlenwasserstoffe wie Methylenchlorid oder THF, z.B. zu Dioxan als Lösungsmittel können zur besseren Solvolyse bei tiefen Temperaturen, z.B. unter ca. 10°C, hinzugesetzt werden.

Für das erfindungsgemäße Verfahren besonders geeignete Ester sind Ester aus niedermolekularen aliphatischen Carbonsäuren, insbesondere Monocarbonsäuren, und niedermolekularen aliphatischen Alkoholen, wobei der Begriff niedermolekular jeweils etwa 1 bis 6 Kohlenstoffatome enthaltend bedeutet. Beispielsweise seien folgende Ester aufgeführt: Essigsäureethylester, Ameisensäureethylester, Propionsäuremethylester, Buttersäuremethylester, Isobuttersäuremethyl- oder -ethylester, wobei Essigester bevorzugt ist.

Die Lösungsmittelmengen sind nicht besonders kritisch, und in weiten Grenzen variierbar. Sie liegen im allgemeinen bei ca. 1 bis 50 Gew.%, insbesondere 2,5 bis 10 Gew.%, bezogen auf das Chlorhydrin II. Höhere Überschüsse an Lösungsmittel sind durchaus möglich, auch können Gemische der z.B. in den Ansprüchen 1 bis 5 genannten Lösungsmittel für die Dehydratisierung verwendet werden, wobei die Mischungsverhältnisse in einem weiten Bereich von ca. 10:1 bis 1:10 variiert werden können. zur Erzielung höherer Raum-Zeit-Ausbeuten und hoher Z-Produktanteile haben sich Zusätze von 5 bis 20 Gew.%, bezogen auf Dioxan, bewährt.

Als wasseraufnehmendes Mittel wird dem Reaktionsgemisch ein Carbonsäureanhydrid zugesetzt. Dabei kommen insbesondere Anhydride aliphatischer niedermolekularer Monocarbonsäuren wie Acetanhydrid, Propionsäureanhydrid, Buttersäureanhydrid und Isobuttersäureanhydrid in Betracht. Es können aber auch Anhydride aliphatischer oder aromatischer Dicarbonsäuren wie Malonsäureanhydrid, Maleinsäureanhydrid, Bernsteinsäureanhydrid oder Phthalsäureanhydrid zugegen sein.

Zur Dehydratisierung werden in der Regel 0,5 bis 3, insbesondere 1 bis 2 Moläquivalente Anhydrid, bezogen auf das Chlorhydrin II verwendet. Größere Mengen sind möglich, bringen aber keine weiteren Vorteile.

Besonders vorteilhafte Ergebnisse erhält man bei einer Kombination aus Dioxan und/oder THF als Lösungsmittel mit Acetanhydrid und Schwefelsäure oder bei Verwendung von Essigsäureethyester als Lösungsmittel mit Isobuttersäureanhydrid und Schwefelsäure.

Die Dehydratisierung wird unter sauren Reaktionsbedingungen durchgeführt, wobei hierfür übliche Säuren, z.B. organische Sufonsäuren wie Trifluormethansulfonsäure, Methansulfonsäure, para-Toluolsulfonsäure oder Naphthalinsulfonsäure und insbesondere konzentrierte Mineralsäuren wie Perchlorsäure, Phosphorsäure und insbesondere Schwefelsäure von 30 bis 99,9 %, vorzugsweise 50 bis 99 oder Oleum. Bei Verwendung von stärker wasserhaltigen Säuren wird allgemein mehr Carbonsäureanhydrid verwendet.

Die Säure kann in katalytischer, in stöchiometrischer Menge oder im Überschuß, bezogen auf II eingesetzt werden. Bevorzugt sind Mengen von etwa 0,01 bis 4 Moläquivalenten, bezogen auf II. Bei Verwendung von Oleum sind geringere Mengen von 0,05 bis 1 Moläquivalenten, bezogen auf II, vorteilhaft.

Eine vorteilhafte Variante des erfindungsgemäßen Verfahrens besteht darin, daß man anstelle des Carbonsäureanhydrids als wasseraufnehmendes Mittel Keten gegebenenfalls in Kombination mit stöchiometrischen oder katalytischen Mengen einer aliphatischen Carbonsäure, bezogen auf II, verwendet. In diesem Fall wird vorteilhaft die Carbonsäure, z.B. eine der oben aufgeführten niedermolekularen aliphatischen Carbonsäuren, vorgelegt und das gasförmige Keten dem Reaktionsgemisch hinzugefügt oder das Keten ohne Carbonsäurezusatz zu dem im Lösungsmittel gelösten Chlorhydrin II gasförmig hinzugegeben. Die Menge an Keten entspricht dabei den oben angegebenen Mengen für das Carbonsäureanhydrid.

Zur Erzielung hoher Z-Isomerenanteile sollte die Dehydratisierung bei möglichst tiefen Temperaturen, d.h. Temperaturen bis etwa 50°C, vorteilhaft -25 bis +40°C, insbesondere -25 bis +30°C, durchgeführt werden.

In der Regel wird die Dehydratisierung bei Normaldruck durchgeführt. Eine Reaktionsführung bei Unter- oder Überdruck ist ebenfalls möglich und eine Druckerhöhung kann in manchen Fällen zu einer Erhöhung der Raum-Zeit-Ausbeute führen.

Die nach dem erfindungsgemäßen Verfahren herstellbaren Z-1,2-Diarylallylchloride der Formel I

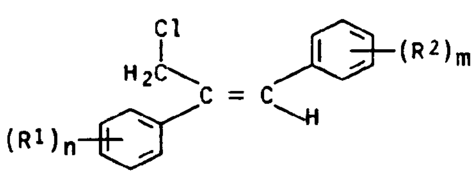

(I),

in der die Reste $R^1$ und $R^2$ unabhängig voneinander Wasserstoff, Halogen, $C_1$-$C_7$-Alkyl, $C_1$-$C_6$-Halogenalkyl, $C_1$-$C_5$-Alkoxy, $C_1$-$C_5$-Halogenalkoxy oder einen unsubstituierten oder ein- bis dreifach durch die für $R^1$ und $R^2$ genannten Reste substituierten aromatischen Rest bedeuten und n für 1, 2 oder 3 steht, sind ebenfalls Gegenstand der Erfindung.

In der Formel I stehen die Indices m und n bevorzugt für 1 und die Substituenten $R^1$ und $R^2$ unabhängig voneinander insbesondere für:

Wasserstoff;

Halogen wie Fluor, Chlor, Brom und Jod, vorzugsweise Chlor und Fluor;

verzweigtes oder unverzweigtes $C_1$-$C_7$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

$C_1$-$C_6$-Halogenalkyl wie Fluormethyl, Difluormethyl, Trifluormethyl, Chloridifluormethyl, Dichlorfluormethyl, Trichlormethyl, 1-Fluorethyl, 2-Fluorethyl, 2,2-Difluorethyl, 2,2,2-Trifluorethyl, 2-Chlor-2, 2-difluorethyl, 2,2-Dichlor-2-fluorethyl, 2, 2, 2-Trichlorethyl und Pentafluorethyl, vorzugsweise Trifluormethyl;

$C_1$-$C_5$-Alkoxy wie Methoxy, Ethoxy, Propoxy, 1-Methylethoxy, Butoxy, 1-Methylpropoxy, 2-Methylpropoxy und 1,1-Dimethylethoxy, vorzugsweise Methoxy, Ethoxy und Propoxy;

$C_1$-$C_5$-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2, 2, 2-Trifluorethoxy, 2-Chlor-1,1,2-trifluorethoxy und Pentafluorethoxy, vorzugsweise Trifluormethoxy;

einen aromatischen Rest, z.B. einen Phenylrest, der unsubstituiert oder ein- bis dreifach substituiert ist, durch einen Rest $R^3$, der die für $R^1$ oder $R^2$ bevorzugt genannte Bedeutung hat, d.h. für Wasserstoff, Halogen eine verzweigte oder unverzweigte $C_1$-$C_7$-Alkylgruppe, eine $C_1$-$C_6$-Halogenalkylgruppe, eine $C_1$-$C_5$-Alkoxygruppe oder eine $C_1$-$C_5$-Halogenalkoxygruppe steht.

Die Reste $R^1$ = 4-F und $R^2$ = 2-Cl werden bevorzugt.

Z-1,2-Diaryl-allylchloride der allgemeinen Formel I besitzen gegenüber den aus DE-A 32 18 129 bekannten 1,2-Diaryl-allylbromiden unerwartete Vorteile. Neben einer sehr einfachen Epoxidierung zu den Diaryl-oxiranen der allgemeinen Formel V ist besonders vorteilig zu nennen, daß man aufgrund einer stereoselektiven Epoxidation keine Isomerengemische der Oxirane erhält, was ausgehend von den bekannten Z-1,2-Diarylallylbromiden der Fall ist, sondern solche, in denen die Arylreste transoid angeordnet sind.

Beispielsweise können die in der folgenden Tabelle 1 aufgeführten Substitutionsmuster vorliegen:

Tabelle 1

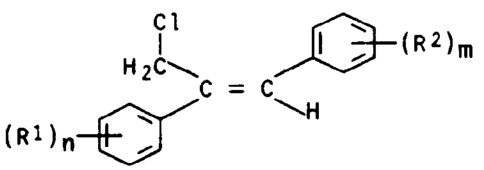

(I),

| Verbindung Nr. | $(R^1)_n$ | $(R^2)_m$ | Schmp. [°C] $^1$H-NMR [ppm] |
|---|---|---|---|
| 1.1 | 3-Cl | 3-Cl | |
| 1.2 | 4-Cl | 2,4-diCl | |
| 1.3 | 4-F | $2-CH_3$ | |
| 1.4 | 4-F | $2-CF_3$ | |
| 1.5 | H | $2-OCF_3$ | |
| 1.6 | 4-F | 2-Cl | 66 |
| 1.7 | $4-OCH_3$ | 2-Cl | |
| 1.8 | 4-Br | 2,4-diCl | |
| 1.9 | $4-C_6H_5-CH_2O$ | $3-CH_3$ | |
| 1.10 | $4-p-ClC_6H_4$ | 2-Cl | |
| 1.11 | $n-C_4H_9$ | 2-Cl | |
| 1.12 | $4-C_6H_5$ | 2,4-diCl | |
| 1.13 | 4-F | $3-CF_3$ | |
| 1.14 | 4,5-diCl | $2-CH_3$ | |
| 1.15 | $4-C_6H_5O$ | 2-Cl | |
| 1.16 | 4-Cl | 2-Cl | 79-82 |

In den Diarylallylchloriden I kann die Bestimmung der Z:E-Isomere in bekannter Weise, z.B. durch HPLC (Hochdruckflüssigkeitchromatographie), gaschromatographisch oder durch $^1$H-NMR-Untersuchungsmethoden unter Verwendung der reinen Z- bzw. E-Isomere als Vergleichsmittel und Standardisierung der entsprechenden Mischungsverhältnisse bestimmt werden.

Die Herstellung der fungiziden Wirkstoffe III und IV, ausgehend von den Diarylallylchloriden I bzw. den Chlorhydrinen II ist im folgenden Reaktionsschema dargestellt:

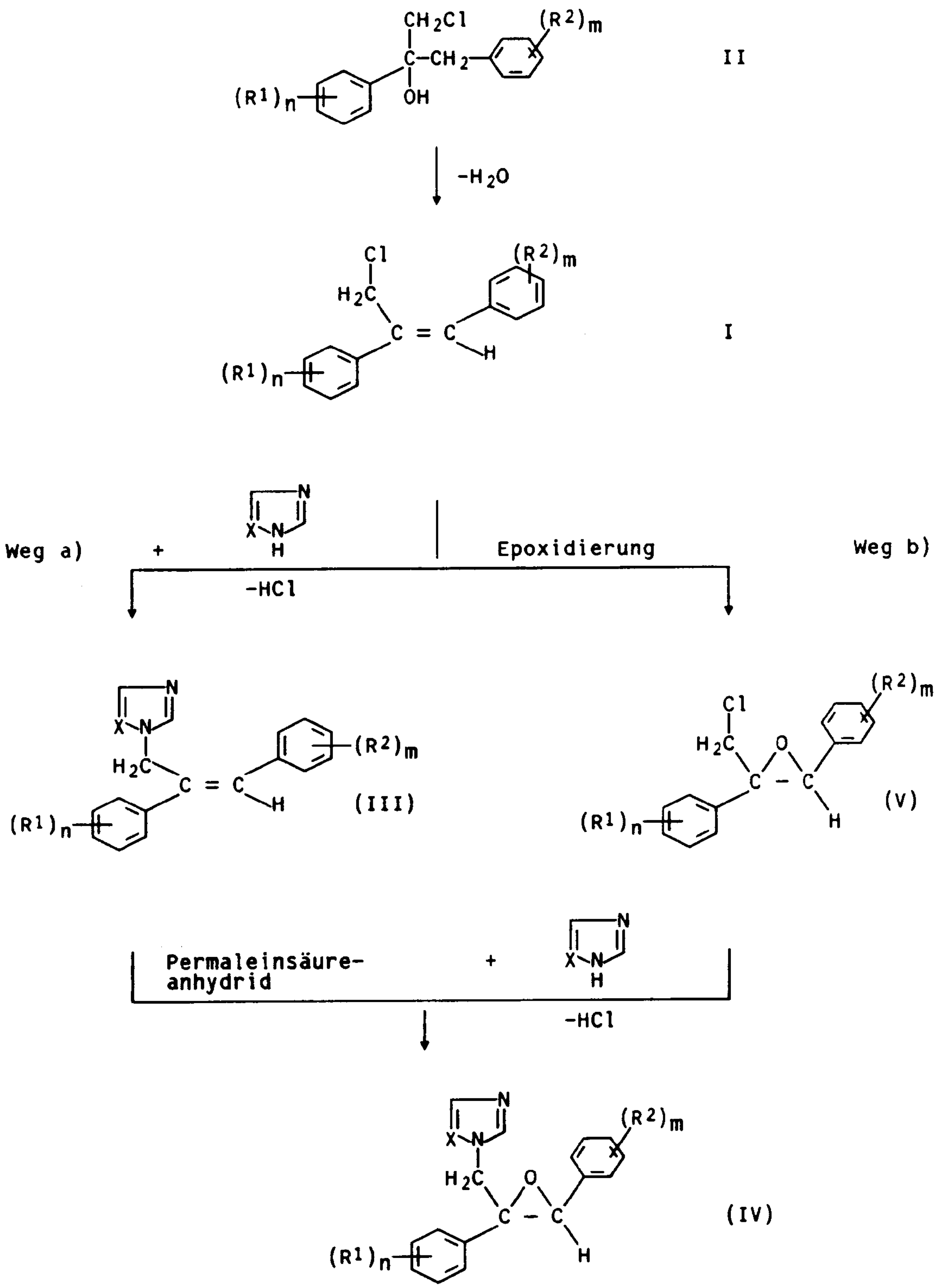


Die Reaktionssequenz gemäß Weg b) kann in an sich bekannter Weise, z.B. wie in DE-A 32 18 129 prinzipiell beschrieben, durchgeführt werden. Die Substitution des Chloratoms durch die Azol- bzw. Imidazolgruppe in Verbindung V wird üblicherweise in einem inerten Lösungsmittel wie Dimethylformamid oder N-Methylpyrrolidon in Gegenwart einer anorganischen oder organischen Base, wie z.B. Natriumhydroxid, Kaliumhydroxid, Natriumcarbonat, Kaliumcarbonat oder Dicyclohexylamin sowie Dimethylcyclohexylamin, durchgeführt.

Die Zwischenprodukte V sind neu. Hinsichtlich der bevorzugten Reste $R^1$ und $R^2$ sowie der Indices n und m gelten die im Fall der Verbindungen I dargelegten Definitionen analog. Beispielsweise können die in der folgenden Tabelle 2 aufgeführten Substitutionsmuster vorliegen:

Tabelle 2

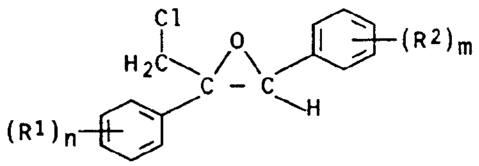

(V),

| Verbindung Nr. | $(R^1)_n$ | $(R^2)_m$ | Schmp. [°C] $^1H$-NMR [ppm] |
|---|---|---|---|
| 2.1 | 3-Cl | 3-Cl | |
| 2.2 | 4-Cl | 2,4-diCl | |
| 2.3 | 4-F | $2-CH_3$ | |
| 2.4 | 4-F | $2-CF_3$ | |
| 2.5 | H | $2-OCF_3$ | |
| 2.6 | 4-F | 2-Cl | 68-70 |
| 2.7 | $4-OCH_3$ | 2-Cl | |
| 2.8 | 4-Br | 2,4-diCl | |
| 2.9 | $4-C_6H_5-CH_2O$ | $3-CH_3$ | |
| 2.10 | $4-p-ClC_6H_4$ | 2-Cl | |
| 2.11 | $n-C_4H_9$ | 2-Cl | |
| 2.12 | $4-C_6H_5$ | 2,4-diCl | |
| 2.13 | 4-F | $3-CF_3$ | |
| 2.14 | 4,5-diCl | $2-CH_3$ | |
| 2.15 | $4-C_6H_5O$ | 2-Cl | |
| 2.16 | 4-Cl | 2-Cl | |

Im Fall des Weges a) verläuft die erste Stufe, d.h. die Substitution, analog der letzten Stufe des Weges b). Vorteilhaft kann die Dehydratisierung und die nachfolgende Substitution im Eintopfverfahren ohne Isolierung und Reinigung der Zwischenstufe II durchgeführt werden.

Die Epoxidierung der Verbindungen III erfolgt erfindungsgemäß in der Weise, daß man in Gegenwart eines hohen Überschusses an Permaleinsäure arbeitet und die Permaleinsäure in situ aus 5 bis 30, insbesondere 5 bis 10 Moläquivalenten Maleinsäureanhydrid, bezogen auf III, und weniger als stöchiometrische Mengen an Wasserstoffperoxidlösung, bezogen auf das Maleinsäureanhydrid umsetzt. Im allgemeinen werden Molverhältnisse von Anhydrid zu $H_2O_2$ von 1,5 bis 10, insbesondere 2 bis 4 eingesetzt. Vorteilhaft kann eine 30 bis 50 %ige wäßrige Lösung von Wasserstoffperoxid verwendet werden.

Die Reaktionstemperatur für die Epoxidierung kann 0 bis 100°C, insbesondere 20 bis 80°C betragen.

Die Epoxidierung wird in Gegenwart eines aprotisch-polaren Lösungsmittels durchgeführt werden. Als Lösungsmittel können z.B. halogenierte Kohlenwasserstoffe wie Dichlormethan, Dichlorethan, Chlorbenzol oder Chlortoluol oder aromatische Kohlenwasserstoffe wie Benzol, Toluol oder Xylol verwendet werden. Die Lösungsmittelmenge ist nicht besonders kritisch und liegt im allgemeinen bei 5 bis 50, insbesondere 10 bis 20 Gew.%, bezogen auf das Olefin.

Nach dieser Epoxidierungsmethode können die Azolylmethyloxirane IV in weitaus höheren Ausbeuten erhalten werden, als nach den in DE-A 32 18 129 beschriebenen Verfahren.

Die einzelnen Syntheseschritte sind in den nachfolgenden Versuchsbeispielen erläutert.

Beispiel 1

Herstellung der Ausgangsstoffe II

1-Chlor-2-(4-chlorphenyl)-3-(2-chlorphenyl)propan-2-ol

9,7 g (0,404 mol) Magnesiumspäne in 20 ml absolutem Ether werden bei 24 bis 36°C binnen 5 min. mit 5,0 g (0,031 mol) 2-Chlorbenzylchlorid versetzt. Nach Anspringen der Reaktion wird eine Lösung von 200 ml absolutem Ether und 50,2 g (0,31 mol) 2-Chlorbenzylchlorid zugetropft. Anschließend wird noch ca. 10 Minuten refluxiert. Unter Stickstoff wird vom überschüssigen Magnesium abdekantiert und die Grignard-Lösung bei 0°C vorgelegt. Danach tropft man 55,7 g (0,3 mol) para-Chlor-ω-chloracetophenon gelöst in 350 ml Toluol hinzu und rührt bei 0°C noch 1,5 Stunden nach. Das Reaktionsgemisch wird bei ca. 2 bis 6°C zu 1,5 l konzentrierter Ammoniumchloridlösung getropft. Nach Extraktion mit Methyl-tert.-butylether und anschließender üblicher Aufarbeitung erhält man 92,9 g (Ausbeute 99 %, Reinheit nach HPLC: 68,2 %) 1-chlor-2-(4-chlorphenyl)-3-(2-chlorphenyl)propan-2-ol als rohes Öl, das direkt weiter umgesetzt werden kann. Zur Charakterisierung wurde aus n-Hexan umkristallisiert.
Schmelzpunkt: 64 bis 69°C.

Beispiele 2 bis 5 und Vergleichsbeispiele I bis IV

Dehydratisierung der Chlorhydrine II

Z-3-Chlor-2-(4-chlorphenyl)-1-(2-chlorphenyl)propen (Verbindung Nr. 1.16 in Tabelle 1)

60 g (0,2 mol) des in Beispiel 1 beschriebenen Chloralkohols werden bei -2°C in 230 ml Dioxan und 23 ml Tetrahydrofuran mit 24,5 g (0,24 mol) Acetanhydrid versetzt und anschließend 2,36 g (0,024 mol) konzentrierte Schwefelsäure zugetropft. Nach 3 Stunden Rühren bei 0°C ist nach HPLC-Analyse praktisch alles Ausgangsmaterial umgesetzt.

Anschließend werden bei 0°C innerhalb von 30 Minuten eine Mischung aus halbgesättigter Natriumchloridlösung und 50 %iger Natronlauge zugegeben, so daß sich ein pH-Wert von 8 bis 9 einstellt.

Zuletzt wird die organische Phase getrocknet und im Vakuum eingeengt und kann ohne weitere Reinigung für Folgeumsetzungen verwendet werden.

Ausbeute 55,7 g (Z/E=9,1/1), rohes Öl, Umkristallisation zum reinen Z-Isomer aus n-Hexan mit Schmelzpunkt 79 bis 82°C.

In analoger Weise können die Z-1,2-Diaryl-allylchloride nach Tabelle 1 hergestellt werden.

Z-3-Chlor-2-(4-fluorphenyl)-1-(2-chlorphenyl)propen (Beispiel Nr. 1.6 in Tabelle 1)

1-Chlor-2-(4-fluorphenyl)-3-(2-chlorphenyl)propan-2-ol, hergestellt durch Grignardaddition von 2-Chlorbenzylmagnesiumchlorid an para-Fluor-ω-chloracetophenon und als Rohmaterial mit einer HPLC-Reinheit von 78-87 % eingesetzt, wurde wie in Beispiel 2 beschrieben unter den in Tabelle 2 angegebenen Reaktionsbedingungen umgesetzt. Der Anteil an Z- bzw. E-Isomer wurde durch HPLC (Hochdruckflüssigkeitschromatographie)-Analyse (nichtkorrigierte relative Flächenprozente) ermittelt.

Tabelle 2: Dehydratisierung von 1-Chlor-2-(4-fluorphenyl)propan-2-ol

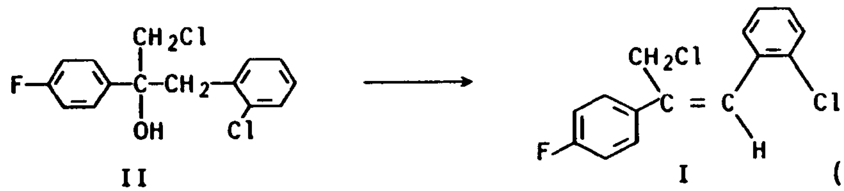

II I (Z-Konfiguration)

| Beispiel | Lösungsmittel | saure Reagenzien | Menge II (g/mol) | Temp. (°C) | Zeit (min) | Ausbeute an Z-I (%) | Verhältnis Z/E | Literatur[a)] |
|---|---|---|---|---|---|---|---|---|
| 3 | 20 ml Dioxan<br>2 ml THF | 0,2 g konz. $H_2SO_4$<br>2,3 g Acetanhydrid | 5/0,018 | -2 | 60 | 58 | 9,2 | |
| 4 | 20 ml Dioxan<br>2 ml THF | 0,2 g konz. $H_2SO_4$<br>2,3 g Acetanhydrid | 5/0,018 | 25 | 30 | 55 | 6,5 | |
| 5 | 20 ml Essigester | 0,2 g konz. $H_2SO_4$<br>3 g Isobutter-säureanhydrid | 5/0,018 | 25 | 30 | 50 | 7,7 | |
| Vgl.bsp. I | 20 ml Dioxan<br>2 ml THF | 0,2 g konz. $H_2SO_4$<br>oder<br>2,3 g Acetanhydrid | 5/0,018 | 25 | 60 | kein Umsatz | | |
| II | 20 ml Essigester<br>2 ml THF | 0,2 g konz. $H_2SO_4$<br>2,3 g Acetylchlorid | 5/0,018 | 40 | 30 | 23[b)] | 6,8 | Ann.chim.et phys. [11] 6,313 (1936) |

Tabelle 2 (Fortsetzung)

| Beispiel | Lösungsmittel | saure Reagenzien | Menge II (g/mol) | Temp. (°C) | Zeit (min) | Ausbeute an Z-I (%) | Verhältnis Z/E | Literatur[a)] |
|---|---|---|---|---|---|---|---|---|
| III | 50 ml Ameisensäure | | 10/0,036 | 100 | 50 | 8,3 | 3,8 | J.Am.Chem. Soc.2204 u. 2208 (1938) |
| IV | 50 g Toluol<br>80 ml Cyclohexan | 2 g p-Toluol-sulfonsäure | 50/0,18.. | Rück-fluß | 150 | 46,5 | 4,5 | Naturwiss. 44, 584 (1957) |
| V | 15 g Acetonitril | 75 mg p-Toluol-sulfonsäure | $2,1/7,56\cdot 10^{-3}$ | 50 | 180 | kein Umsatz | | Tetrahedron 26, 4277-4286 (1970) |

a) angelehnt an die genannten Literaturstellen
b) 19 % Edukt, 13 % Acylatbildung über die OH-Funktion im Chlorhydrin II

Beispiel 6

Herstellung des Chlorhydrins und in situ-Dehydratisierung

1-Chlor-2-(4-fluorphenyl)-3-(2-chlorphenyl)propan-2-ol

36,0 g (1,5 mol) Magnesiumspäne wurden in 200 ml Diethylether vorgelegt und 170 g (1,0 mol) 2-Chlorbenzylchlorid gelöst in 400 ml Diethylether zugetropft. Anschließend wurden 155 g (0,9 mol) para-Fluor-ω-chloracetophenon, gelöst in 450 ml Diethylether, bei -10°C zugetropft. Danach wird bei 25°C noch 2 Stunden nachgerührt.

Nun wird 49,0 g (0,5 mol) konzentrierter Schwefelsäure in 300 ml Diethylether bei -10°C zugetropft. Man läßt auf 25°C erwärmen und saugt vom ausgefallenen Salz ab. Die rohe etherische Lösung des Chlorhydrins wird anschießend weiter eingesetzt.

Z-3-Chlor-2-(4-fluorphenyl)-1-(2-chlorphenyl)propen

525 ml der oben beschriebenen rohen Lösung, mit ca. 134,5 g Chlorhydrin (entsprechend 0,45 mol), werden mit 8,0 g (0,08 mol) konzentrierter Schwefelsäure bei -10°C versetzt, und anschließend werden 57,1 g (0,56 mol) Acetanhydrid binnen 2 Stunden zugetropft. Danach wird erneut von etwas ausgefallenen Salz abfiltriert. Nach Abdampfen des Lösungsmittels aus dem Filtrat kann das rohe Allylchlorid für die Triazolsubstitution bzw. für die Epoxidierung weiter verwendet werden.

Beispiel 7

Keten-Variante

Z-3-Chlor-2-(4-fluorphenyl)-1-(2-chlorphenyl)propen

Bei 0°C werden 250 ml Dioxan, 25 ml Tetrahydrofuran, 12,4 g Essigsäure (0,2 mol) und 69 g (0,23 mol) aus der Grignard-Reaktion gemäß Beispiel 1 erhaltenes rohes 1-Chlor-2-(4-fluorphenyl)-3-(2-chlorphenyl)propan-2-ol vorgelegt und binnen ca. 1 Stunde 43 g (1,02 mol) Keten eingegast. Nach üblicher Aufarbeitung wurde nach HPLC-Analyse eine praktisch identische Ausbeute wie bei Verwendung von Acetanhydrid im oben beschriebenen Beispiel 2 erreicht. Die Z/E-Isomerenanteile betragen für diese Reaktionsführung ca. 11:1.

Beispiele 8 und 9

Herstellung der Azolylmethyloxirane IV gemäß Weg a)

Z-3-(1,2,4-Triazol-1-yl)-2-(4-chlorphenyl)-1-(2-chlorphenyl)propen

Eine Lösung von 11,5 g (0,17 mol) Triazol in 150 ml Dimethylformamid wird mit 6,6 g Natriumhydroxid versetz und auf ca. 70°C erwärmt bis unter Rühren eine klare Lösung entstanden ist. Anschließend wird auf 10°C abgekühlt und 49,5 g des gemäß Beispiel 2 hergestellten Z-3-Chlor-2-(4-chlorphenyl)-1-(2-chlorphenyl) propen als Rohprodukt gelöst in 50 ml Dimethylformamid binnen 1 Stunde zugetropft und danach noch 4 Stunden bei Raumtemperatur nachgerührt.

Anschließend werden 200 ml Wasser zugegeben und mehrfach mit Methyl-tert.butylether extrahiert. Die vereinigten organischen Phasen werden gewaschen, getrocknet und im Vakuum eingeengt. Man erhält durch Umkristallisieren aus Methyl-tert.-butylether und n-Hexan 24,4 g Z-3-(1,2,4-Triazol-1-yl)-2-(4-chlorphenyl)-1-(2-chlorphenyl)propen mit dem Schmelzpunkt 106-110°C.

cis-2-(1,2,4-Triazol-1-ylmethyl)-2-(4-fluorphenyl)-3-(2-chlorphenyl)oxiran

84 g (0,9 mol) Maleinsäureanhydrid und 6 Tropfen konzentrierte Schwefelsäure werden in 90 ml Dichlorethan mit 22 g 50 %igem Wasserstoffperoxid auf 50°C erwärmt und tropfenweise mit 28 g (0,089 mol) z-3-(1,2,4-Triazol-1-yl)-2-(4-fluorphenyl)1-(2-chlorphenyl)propen in 75 ml Dichlorethan versetzt. Man rührt 3 Stunden bei dieser Temperatur nach und anschließend noch 2,5 Stunden bei 70°C.

Nach dem Abkühlen des Reaktionsgemisches wird von ausgefallener Maleinsäure abgesaugt und mit Thiosulfatlösung und verdünnter Natronlauge ausgeschüttelt. Die getrocknete und im Vakuum bei etwa 50°C weitgehend eingedampfte organische Phase liefert nach Abkühlen und erneutem Einengen der Mutterlauge 14 g Wertprodukt (≙ 50 % Ausbeute).

Beispiel 10 und 11

Herstellung der Azolylmethyloxirane IV gemäß Weg b)

cis-1-Chlormethyl-2-(2-chlorphenyl)-1-(4-fluorphenyl)oxiran (Verbindung Nr. 2.6 in Tabelle 2)

56,2 g (0,2 mol) Z-3-Chlor-2-(4-fluorphenyl)-1-(2-chlorphenyl)propen werden in 530 ml Eisessig mit 196 g (2 mol) Maleinsäureanhydrid vorgelegt und bei 25°C binnen einer Stunde mit 68 g (1 mol) 50 %iger Wasserstoffperoxidlösung versetzt. Man rührt noch 3 bis 4 Stunden bei 40°C und anschließend noch 10 Stunden bei 25°C nach.

Zuletzt wird die Reaktionsmischung in 3 Liter Wasser und 50 ml 10 %iger Natriumthiosulfatlösung eingerührt und gegebenenfalls erneut mit etwas Thiosulfatlösung versetzt, bis kein Peroxid mehr nachzuweisen ist. Das dabei anfallende farblose Präzipitat wird abgesaugt und getrocknet. Das Rohmaterial wurde ohne weitere Reinigung weiter eingesetzt.
(Umkristallisation aus n-Hexan; Fp. 68 bis 70°C).

cis-2-(1,2,4-Triazol-1-ylmethyl)-2-(4-fluorphenyl)-3-(2-chlorphenyl)oxiran

1,5 g (5 mmol) Cis-1-Chlormethyl-2-(2-chlorphenyl)-1-(4-fluorphenyl)oxiran und 0,69 g (7,5 mmol) Natrium-1,2,4-Triazolid werden 5 Stunden in 7 ml Dimethylformamid bei 75°C gerührt. Nach dem Abkühlen neutralisiert man durch Zugabe von etwas Essigsäure und versetzt mit wenig Wasser (ca. 10 ml), wobei kristallines Produkt ausfällt (Ausbeute: 1,4 g). Man saugt ab, wäscht mit Wasser nach und trocknet im Vakuum.

## Patentansprüche

1. Verfahren zur stereoselektiven Herstellung von Z-1,2-Diaryl-allylchloriden der allgemeinen Formel I

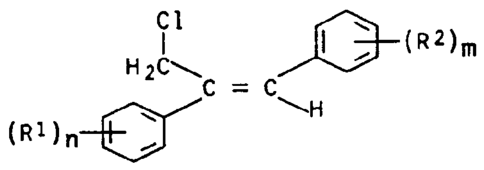

(I),

in der die Reste $R^1$ und $R^2$ unabhängig voneinander Wasserstoff, Halogen, Alkyl, Halogenalkyl, Alkoxi, Halogenalkoxi oder einen unsubstituierten oder substituierten aromatischen Rest bedeuten und n und m für 1, 2 oder 3 stehen, dadurch gekennzeichnet, daß man Chlorhydrine der Formel II

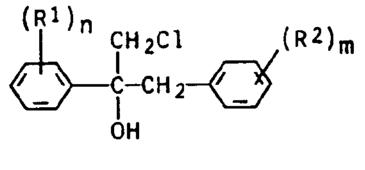

(II),

in der die Reste die o.g. Bedeutung haben, in einem inerten Ether oder Carbonsäureester als Lösungsmittel in Gegenwart eines Carbonsäureanhydrids und einer organischen oder anorganischen Säure bei Temperaturen bis 50°C dehydratisiert.

2. Verfahren nach Anspruch 1, dadurch gekennzeichnet, daß man anstelle des Carbonsäureanhydrids Keten gegebenenfalls in Kombination mit einer katalytischen bis stöchiometrischen Menge einer organischen Carbonsäure, bezogen auf das Chlorhydrin II, verwendet.

3. Verfahren nach Anspruch 1, dadurch gekennzeichnet, daß man die Dehydratisierung bei Temperaturen von -25 bis +30°C vornimmt.

4. Verfahren nach Anspruch 1, dadurch gekennzeichnet, daß man die Dehydratisierung in Gegenwart von 0,01 bis 4 Moläquivalenten Schwefelsäure und 0,5 bis 3 Moläquivalenten Carbonsäureanhydrid durchführt.

5. Verfahren nach Anspruch 1, dadurch gekennzeichnet, daß man die Dehydratisierung in Gegenwart von 0,05 bis 1 Moläquivalenten Oleum im Gemisch mit 1 bis 2 Moläquivalenten Carbonsäureanhydrid durchführt.

6. Verfahren zur Herstellung von Azolylmethyloxiranen der Formel IV

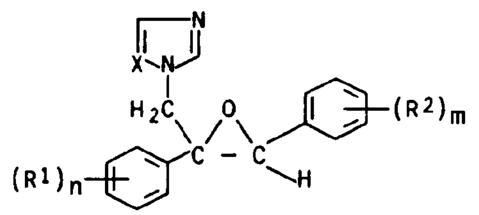

(IV),

in der die Reste $(R^1)_n$ und $(R^2)_m$ die in Anspruch 1 genannte Bedeutung haben und X für CH oder N steht, dadurch gekennzeichnet, daß man die Z-1,2-Diarylallylchloride I gemäß Anspruch 1

a) mit 1,2,4-Triazol oder Imidazol in Gegenwart einer Base zu Z-1,2-Diaryl-allyltriazolen bzw. -imidazolen der Formel III

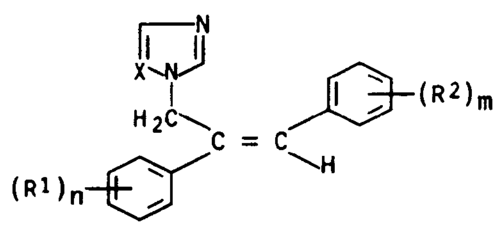

(III),

in der $(R^1)_n$, $(R^2)_m$ und X die oben genannte Bedeutung haben, umsetzt und anschließend die Verbindung III in einem polaren aprotischen Lösungsmittel mit Permaleinsäure, in situ hergestellt aus 5 bis 15 Moläquivalenten Maleinsäureanhydrid, bezogen auf Verbindung III und unterstöchiometrischen Mengen an Wasserstoffperoxidlösung, bezogen auf das Maleinsäureanhydrid, zu den Azolylmethyloxiranen I umsetzt oder

b) in üblicher Weise zu Chlormethyl-diaryl-oxiranen der Formel V epoxidiert

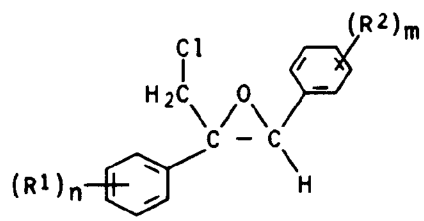

(V)

und anschließend mit 1,2,4-Triazol oder Imidazol in Gegenwart einer Base zu den Azolylmethyloxiranen IV umsetzt.

7. Verfahren zur stereoselektiven Herstellung von Z-1,2-Diaryl-allylchloriden der allgemeinen Formel I gemäß Anspruch 1, dadurch gekennzeichnet, daß man eine Benzylgrignard-Verbindung VI

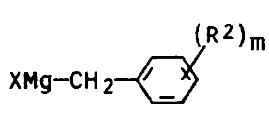

VI

in der X für Chlor und Brom steht in an sich bekannter Weise in Diethylether als Lösungsmittel an ein ω-

Chloracetophenon VII

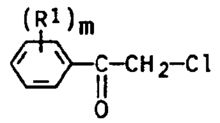

VII

addiert und das so erhaltene Chlorhydrin der Formel II gemäß Anspruch 1 in situ gemäß Anspruch 1 dehydratisiert.

**8.** Z-1,2-Diaryl-allylchloride der Formel I

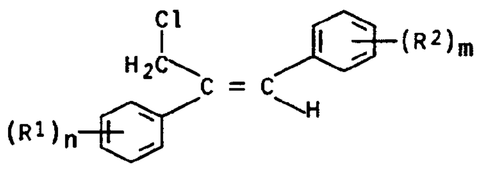

(I),

in der die Reste $R^1$ und $R^2$ unabhängig voneinander Wasserstoff, Halogen, $C_1$-$C_7$-Alkyl, $C_1$-$C_6$-Halogenalkyl, $C_1$-$C_5$-Alkoxy, $C_1$-$C_5$-Halogenalkoxy oder einen unsubstituierten oder ein- bis dreifach durch die für $R^1$ und $R^2$ genannten Reste substituierten aromatischen Rest bedeuten und n für 1, 2 oder 3 steht.

**9.** Chlormethyl-diaryl-oxirane der allgemeinen Formel V

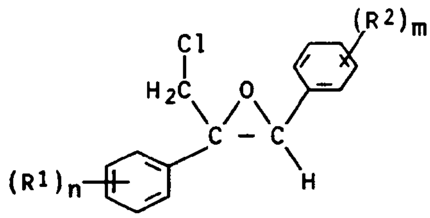

(V)

in der die Reste $R^1$ und $R^2$ unabhängig voneinander Wasserstoff, Halogen, $C_1$-$C_7$-Alkyl, $C_1$-$C_6$-Halogenalkyl, $C_1$-$C_5$-Alkoxy, $C_1$-$C_5$-Halogenalkoxy oder einen unsubstituierten oder ein- bis dreifach durch die für $R^1$ und $R^2$ genannten Reste substituierten aromatischen Rest bedeuten und n für 1, 2 oder 3 steht, mit der Maßgabe, daß $R^2$ keine 2-Methylgruppe darstellt.

## Claims

**1.** A process for the stereoselective preparation of Z-1,2-diarylallyl chlorides of the general formula I

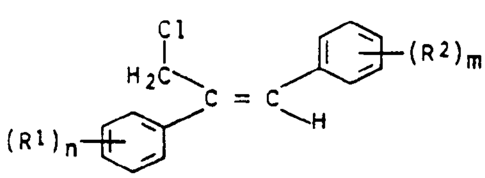

(I),

in which $R^1$ and $R^2$, independently of one another, are hydrogen, halogen, alkyl, haloalkyl, alkoxy, haloalkoxy or an unsubstituted or substituted aromatic radical, and n and m are 1, 2 or 3, which comprises dehydrating a chlorohydrin of the formula II

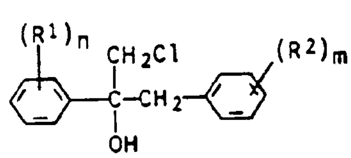

(II),

in which the radicals are as defined above, at up to 50°C in an inert ether or carboxylic acid ester as solvent and in the presence of a carboxylic anhydride and an organic or inorganic acid.

2. The process as claimed in claim 1, wherein ketene if desired in combination with a catalytic to stoichiometric amount of an organic carboxylic acid, based on the chlorohydrin II, is used in place of the carboxylic anhydride.

3. The process as claimed in claim 1, wherein the dehydration is carried out at from -25 to +30°C.

4. The process as claimed in claim 1, wherein the dehydration is carried out in the presence of from 0.01 to 4 mole equivalents of sulfuric acid and from 0.5 to 3 mole equivalents of carboxylic anhydride.

5. The process as claimed in claim 1, wherein the dehydration is carried out in the presence of from 0.05 to 1 mole equivalents of oleum mixed with from 1 to 2 mole equivalents of carboxylic anhydride.

6. A process for the preparation of an azolylmethyloxirane of the formula IV

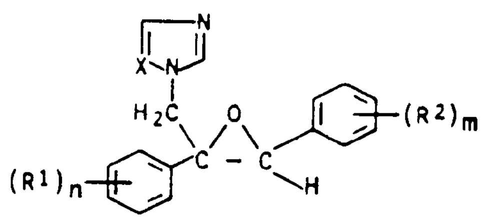 (IV),

in which $(R^1)_n$ and $(R^2)_m$ are as defined in claim 1, and X is CH or N, wherein the Z-1,2-diaryllalyl chloride I as claimed in claim 1

a) is reacted with 1,2,4-triazole or imidazole in the presence of a base to give a Z-1,2-diarylallyl triazole or -imidazole of the formula III

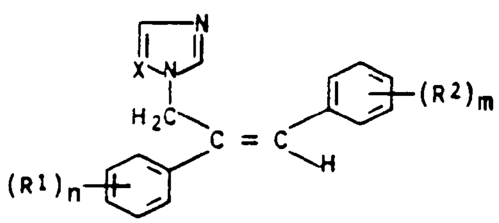 (III),

in which $(R^1)_n$, $(R^2)_m$ and X are as defined above, and the compound III is subsequently reacted in a polar aprotic solvent with permaleic acid, in situ from 5 to 15 mole equivalents of maleic anhydride, based on the compound III, and a substoichiometric amount of hydrogen peroxide solution, based on the maleic anhydride, to give the azolylmethyloxirane I, or

b) is epoxidized in a conventional manner to give a chloromethyldiaryloxirane of the formula V

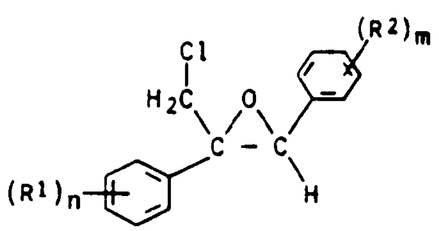 (V)

which is subsequently reacted with 1,2,4-triazole or imidazole in the presence of a base to give an azolylmethyloxirane IV.

7. The process for the stereoselective preparation of a Z-1,2-diarylallyl chloride of the general formula I as claimed in claim 1, wherein a benzyl-Grignard compound VI

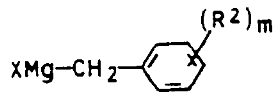

VI

in which X is chlorine or bromine, in diethyl ether as solvent is added in a conventional manner to an ω-chloro-acetophenone VII

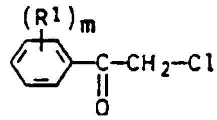

VII

and the chlorohydrin of the formula II as claimed in claim 1 obtained in this way is dehydrated in situ as claimed in claim 1.

**8.** A Z-1,2-diarylallyl chloride of the formula I

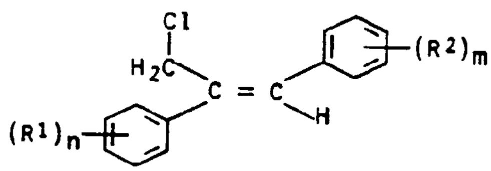

(I),

in which $R^1$ and $R^2$, independently of one another, are hydrogen, halogen, $C_1$-$C_7$-alkyl, $C_1$-$C_6$-haloalkyl, $C_1$-$C_5$-alkoxy, $C_1$-$C_5$-haloalkoxy or an aromatic radical which is unsubstituted or monosubstituted to trisubstituted by the radicals mentioned for $R^1$ and $R^2$, and n is 1, 2 or 3.

**9.** A chloromethyldiaryloxirane of the general formula V

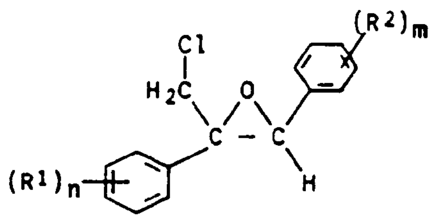

(V)

in which $R^1$ and $R^2$, independently of one another, are hydrogen, halogen, $C_1$-$C_7$-alkyl, $C_1$-$C_6$-haloalkyl, $C_1$-$C_5$-alkoxy, $C_1$-$C_5$-haloalkoxy or an aromatic radical which is unsubstituted or monosubstituted to trisubstituted by the radicals mentioned for $R^1$ and $R^2$, and n is 1, 2 or 3, with the proviso that $R^2$ is not 2-methyl.

## Revendications

**1.** Procédé de préparation stéréosélective de Z-1,2-diarylallyl-chlorures de formule générale I

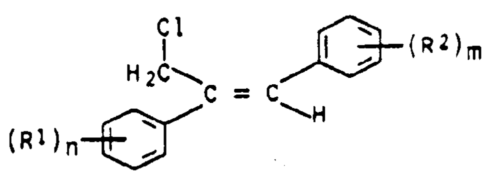

(I),

dans laquelle les restes $R^1$ et $R^2$, indépendamment l'un de l'autre, représentent hydrogène, halogène, alkyle, halogénalkyle, alcoxy, halogénalcoxy ou un reste aromatique non substitué ou substitué, et n et m sont mis pour 1, 2 ou 3, caractérisé par le fait que l'on déshydrate, à des températures allant jusqu'à 50 °C,

des chlorhydrines de formule II

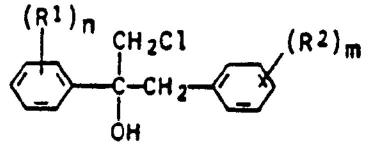

(II),

dans laquelle les restes ont les significations données plus haut, dans un éther ou ester d'acide carboxylique inertes en tant que solvants, en présence d'un anhydride carboxylique et d'un acide organique ou inorganique.

2. Procédé selon la revendication 1, caractérisé par le fait qu'à la place de l'anhydride carboxylique on utilise du cétène, éventuellement en combinaison avec une quantité catalytique à stoechiométrique, rapportée à la chlorhydrine II, d'un acide carboxylique organique.

3. Procédé selon la revendication 1, caractérisé par le fait que l'on effectue la déshydratation à des températures comprises entre -25 et +30°C.

4. Procédé selon la revendication 1, caractérisé par le fait que l'on effectue la déshydratation en présence de 0,01 à 4 équivalents (moles) d'acide sulfurique et 0,5 à 3 équivalents (moles) d'anhydride carboxylique.

5. Procédé selon la revendication 1, caractérisé par le fait que l'on effectue la déshydratation en présence de 0,05 à 1 équivalent (mole) d'oléum en mélange avec 1 à 2 équivalents (moles) d'anhydride carboxylique.

6. Procédé de préparation d'azolylméthyloxiranes de formule IV

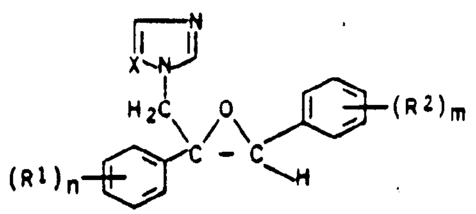

(IV),

dans laquelle les restes $(R^1)_n$ et $(R^2)_m$ ont les significations données dans la revendication 1 et X est mis pour CH ou N, caractérisé par le fait que les Z-1,2-diarylallylchlorures I, selon la revendication 1,

a) sont mis à réagir avec du 1,2,4-triazole ou de l'imidazole, en présence d'une base, pour obtenir des Z-1,2-diaryl-allyltriazoles ou -imidazoles de formule III

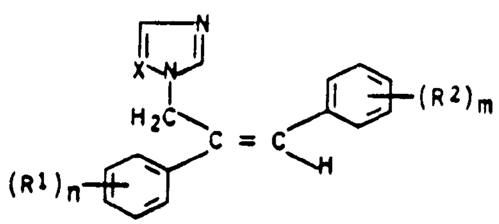

(III),

dans laquelle $(R^1)_n$, $(R^2)_m$ et X ont les significations données et le composé III est ensuite transformé dans un solvant aprotique polaire, en azolylméthyloxiranes I, avec de l'acide permaléique préparé in situ à partir de 5 à 15 équivalents (moles) d'anhydride maléique, rapportés au composé III, et de quantités inférieures aux quantités stoechiométriques (rapportées à l'anhydride maléique) de solution de peroxyde d'hydrogène,

b) ou sont époxydés de manière usuelle en chlorométhyldiaryl-oxiranes de formule V

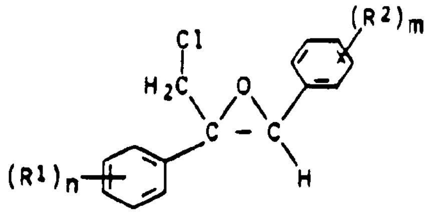

(V)

et sont mis à réagir ensuite, en présence d'une base, avec du 1,2,4-triazole ou de l'imidazole, pour obtenir les azolylméthyloxiranes IV.

7. Procédé de préparation stéréosélective de Z-1,2-diarylallylchlorures de formule générale I, selon la revendication 1, caractérisé par le fait que l'on fixe par addition, de manière en soi connue, dans de l'éther diéthylique comme solvant, un composé de Grignard benzylique VI

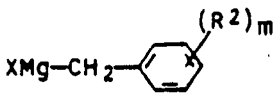

VI

dans laquelle X est mis pour chlore et brome, sur une ω-chloracétophénone VII

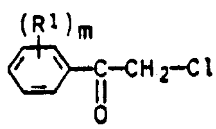

VII

et l'on déshydrate in situ, conformément à la revendication 1, la chlorhydrine résultante de formule II, selon la revendication 1.

8. Z-1,2-diaryl-allylchlorures de formule I

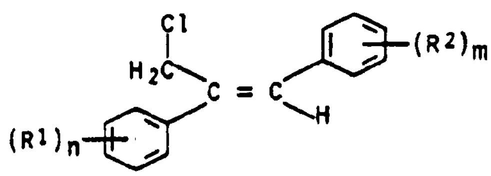

(I),

dans laquelle les restes $R^1$ et $R^2$, indépendamment l'un de l'autre, représentent hydrogène, halogène, alkyle en C1-C7, halogénalkyle en C1-C6, alcoxy en C1-C5, halogénalcoxy en C1-C5 ou un reste aromatique non substitué ou substitué une à trois fois par les restes indiqués pour $R^1$ et $R^2$, n et m étant mis pour 1, 2 ou 3.

9. Chlorométhyl-diaryl-oxiranes de formule générale V

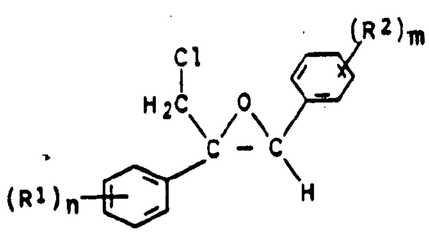

(V)

dans laquelle les restes $R^1$ et $R^2$, indépendamment l'un de l'autre, représentent hydrogène, halogène, alkyle en C1-C7, halogénalkyle en C1-C6, alcoxy en C1-C5, halogénalcoxy en C1-C5 ou un reste aromatique non substitué ou substitué une à trois fois par les restes indiqués pour $R^1$ et $R^2$, et n et m sont mis pour 1, 2 ou 3, étant entendu que $R^2$ ne représente pas un groupe 2-méthyle.